# EUROPEAN PATENT SPECIFICATION

(11) **EP 0 446 262 B1**
(45) Date of publication and mention of the grant of the patent: **16.03.1994**
(21) Application number: 90900227.1
(22) Date of filing: 28.11.1989
(51) Int. Cl.: A61L 27/00, A61K 31/54

(54) **IMPLANT FOR USE IN BONE SURGERY**
IMPLANTAT ZUR VERWENDUNG IN DER KNOCHENCHIRURGIE
IMPLANT UTILISE EN CHIRURGIE OSSEUSE

(30) Priority: 30.11.1988 GB 8827986
(43) Date of publication of application: 18.09.1991
(73) Proprietor: ED. GEISTLICH SÖHNE AG FÜR CHEMISCHE INDUSTRIE, CH-6110 Wolhusen Lucerne (CH)
(72) Inventor: PFIRRMANN, Rolf, Wilhelm, CH-6006 Lucerne (CH)
(74) Representative: Holmes, Michael John
(86) International application number: GB8901423
(87) International publication number: WO9006138

(56) References cited:
- EP-A- 0 048 558
- EP-A- 0 139 534
- WO-A-60/7265
- US-A- 4 789 663

## Description

This invention relates to a novel collagen- based sponge material for use as an implant in bone surgery.

The treatment of osteomyelitis and osteitis is notoriously difficult. It is necessary to remove all infected bone material and then to induce remodell- ing, that is re-growth of healthy bone tissue, within the cavity as formed. Unfortunately, re- infection is common and it is necessary that a powerful antibacterial substance is present during the remodelling phase. Remodelling in facio-maxillary surgery and tooth extraction socket filling imposes similar requirements.

In our European Patent No. 48558 we have described resorbable aqueous gels comprising cross- linked gelatin or collagen materials containing antibiotics such as gentamycin or more preferably, taurolidine, for implantation into osteitis cavities. While such gels have proved successful for most purposes, there is a need for alternative implant materials, particularly for implantation into cavities in small bones such as those of the fingers and toes, as well as tooth extraction cavities and other relatively small cavities.

We have now found that a lyophillised sponge of collagen fibres containing the antibacterial substances taurolidine and/or taurultam provide an extremely effective implant material for such uses. Although collagen sponges have been proposed which contain cross-linked or otherwise pre-treated collagen fibres together with an antibiotic such as gentamycin, it has not previously been proposed to impregnate such sponges with taurolidine and taurultam, which have particular advantages in treating bone cavities and bone injuries generally.

According to the present invention we provide a lyophilised collagen sponge for use as an implant in osteitis and other bone cavities, said sponge having dispersed therein antibacterially effective quantitites of taurolidine and/or taurultam.

Taurolidine and taurultam are methylol transfer agents which are able to combat not only gram negative and gram positive bacteria but also the exotoxins and endotoxins they produce. They are thus particularly well suited for the treatment of bone cavities liable to re-infection. The soluble collagen sponge according to the invention releases the active substances firstly by diffusion and then by dissolution or resorption of the collagen. The sponge conveniently contains from 1-30 mg/cm² of taurolidine and/or from 1-60 mg/cm² taurultam.

Collagen fibres are the most common type of fibres in the connective tissue and the commonest protein in the human body, corresponding to 30% of the total protein. The hyaline cartilage material of the bone consists of 40-45% of collagen fibres. Human bone contains about 40g collagen nitrogen per kg. Collagen fibres consist of collagen fibrils having diameters of 0.2 to 0.5 microns. Their peptide structure contains a high level of proline (12%) and hydroxyproline (10%) residues. Each fibril consists of overlapping molecules of tropocollagen each of which includes a superhelix of 3 polypeptide alpha- chains, which are interwound and stabilized by hydrogen bonding and have terminal non-helical telopeptide sequences.

Four types of collagen are recognised, in which the tropocollagen is built up from three different polypeptide alpha-chains with an average molecular weight of 100,000. The commonest is Type I, occurrring for example in skin, muscle, bone, tendons and fascia, which consists of two identical alpha-1-chains and one alpha-2- chain with a different amino acid sequence. Types II, III, and IV consist of three alpha-1-chains which differ in their primary structure in different parts of the body. Type II is the most common collagen type in the hyaline cartilage. Type III occurs inter alia, in the blood vessels and in foetal membranes. Type IV occurs in the basal laminae.

There are significant differences between collagen fibres at different conditions of maturity. Where the connective tissue is in an active phase of fibrillo-genesis, for example during growth or wound healing, collagen fractions can be isolated with different properties. The first fraction is extractable by neutral solutions (neutral-soluble collagen); this consists of recently synthesized tropocollagen molecules which are not aggregated or are only beginning to aggregate. The second fraction is extractable by a sodium citrate solution at pH 3.0, and is thus termed the acid-soluble collagen fraction. The third fraction found in older tissues is the insoluble fraction and can only be extracted by very vigorous methods. One basis for the difference between these fractions lies in the degree of cross-linking by oxidation to produce peroxide bridges. Collagen can also be cross-linked chemically via free amino groups, using aldehydes such as formaldehyde or glutaraldehyde or isocyanates such as hexamethylene diisocyanate. By such cross-linking, animal collagen fractions lose their antigenicity almost completely. Cross-linking of collagen fibrils in this way is for example, made use of in heart replacement surgery, where animal, e.g. porcine, valves are conditioned with glutaraldehyde for use as human pulmonary or mitral valve replacements.

In general, it is preferred that the collagen is water insoluble but is rapidly resorbed. This is compatible with the relatively short half- life of taurolidine and taurultam. Aged or acid soluble collagen may thus be used or, more preferably, neutral soluble collagen fibres may be artifically aged by oxidation, eg using a peroxide such as hydrogen peroxide, to form oxygen bridges. Collagen of type I, especially from skin and tendons, advantageously from the flank skin of young calves, is preferred.

However, it may be beneficial to lightly cross-limits neutral soluble collagen, eg by treatment with a cross-linking agent, for example an aldehyde such as formaldehyde or glutaraldehyde or a isocyanate such as hexamethylene diisocyanate. Such a cross- linked form of collagen will be resorbed more slowly and thus may release the taurolidine or taurultam over a longer period. It is particularly preferred, however, that the level of any cross-linking is much that the collagen is resorbed in 12 hours or less after implantation.

Where the collagen is cross linked it may be beneficial to include an emulsifying agent, during the foaming and lyophilising step eg lecithin and/or Cremophor EL (available from BASF), both of which are parenterally acceptable.

Suitable collagen sponge may be obtained commercially, for example from Pentapharm AG of Basel, Switzerland, from Dr Otto Suwelak GmbH of Billerbeck, West Germany or from Ed Geistlich Söhne A.G. of Wolhusen, Switzerland. Alternatively, such material may be obtained from the appropriate tissues by conventional methods.

Thus, for example, bovine skin, advantageously from young calves, and preferably from the flank region, may be chemically dehaired and mechanically split to separate off the epidermis and the underskin with associated fat. It is important to avoid or minimize contamination with fat. The layer so obtained may be treated with mild alkali, such as calcium hydroxide, eg for about 4 weeks. The resulting material may then be acidified, eg with 3% hydrochloric acid, washed with running water and comminuted. A proteolytic enzyme may be used to assist separation of collagen from other proteins and a lipase may be used to remove residual fat. However, it is important to avoid antigenic reactions which may result from the use of such enzymes.

The neutral-soluble collagen so produced may then be treated with an oxidising agent such as hydrogen peroxide to form oxygen bridges similar to those formed in the natural ageing of collagen.

The comminuted product may then be homogenised with about 7 parts by weight of water, the pH adjusted to about 5.3 and the product further homogenised to produce a foam.

The foamed homogenised material is then filled into cooling cells, e.g. to a depth of about 1.5 cm, rapidly cooled to -20°C and lyophilised.

The incorporation of the taurolidine or taurultam may be effected either by foaming a collagen solution containing e.g. 2% taurolidine or taurultam, prior to lyophilisation or by redissolving lyophilised collagen in a solution of taurolidine or taurultam and re-lyophilising.

The lyophilised collagen sponge material will normally be sealed in plastic containers and sterilised by radiation e.g. gamma radiation.

Sheets of the collagen sponge according to the invention may be conveniently about 0.5 cm in thickness. Such sheets can be readily cut by the surgeon into small shaped pieces for use as implants. They will normally be laid into the bone cavity without compression. If necessary, spongeosa may also be introduced into the cavity at the same time.

The invention is illustrated by the following non-limiting Examples. Collagen GN is available from Ed. Geistlich Söhne A.G.

### Example 1

Collagen GN (a fleecy material containing some collagen fibres, 21 x 29.8cm = 625.8cm²) is soaked with 260g of a 4.8% (w/w) taurolidine solution and then immediately frozen. Freeze-drying gives a compact taurolidine-collagen sponge with 20 mg taurolidine/cm².

### Example 2

Collagen GN (21 x 29.8 cm = 625.8cm²) is soaked with 260g of a 4.8% (w/w) taurolidine solution, imediately frozen and then freeze-dried. The dried material is soaked a second time with 130g of a 4.8% (w/w) taurolidine solution and freeze-dried to give a compact taurolidine-collagen sponge with 30 mg taurolidine/cm².

### Example 3

Collagen GN (21 x 29.8 cm = 625.8cm²) is soaked with 250g of a 15% taurultam solution and immediately frozen. Freeze-drying gives a compact taurultam-collagen sponge with 60 mg taurultam/cm².

### Example 4

Collagen GN (21 x 29.8 cm = 625.8cm²) is soaked with 287.5g of a 13.05% taurultam solution and immediately frozen. Freeze-drying gives a soft taurultam-collagen sponge with 60 mg taurultam/cm².

### Example 5

Collagen GN (21 x 29.8 cm = 625.8cm²) is soaked with 537.5g of a 7% taurultam solution and immediately frozen. Freeze-drying gives a soft, downy taurultam-collagen sponge with 60 mg taurultam/cm².

## Claims

1. A lyophilised collagen sponge for use as an implant in osteitis and other bone cavities, said sponge having dispersed therein antibacterially effective quantitites of taurolidine and/or taurultam.

2. A collagen sponge as claimed in claim 1 wherein the collagen is selected from acid soluble collagen and artificially aged neutral soluble collagen, whereby the resulting implant is capable of resorption in the human body within up to 12 hours.

3. A collagen sponge as claimed in claim 1 or claim 2 wherein the collagen comprises type I collagen.

4. A process for the preparation of a collagen sponge as defined in claim 1 in which a solution or suspension of collagen fibres in an aqueous solution of taurolidine or taurultam is lyophilised.

5. Use of a collagen sponge as defined in claim 1 in surgery.

## Patentansprüche

1. Lyophilisierter Kollagenschwamm zur Verwendung als Implantat bei Knochenentzündung und in anderen Knochenhöhlen, wobei in dem Schwamm antibakteriell wirksame Mengen an Taurolidin und/oder Taurultam dispergiert sind.

2. Kollagenschwamm nach Anspruch 1, worin das Kollagen aus säurelöslichem Kollagen und künstlich gealtertem, neutrallöslichen Kollagen ausgewählt ist, wobei das so erhaltene Implantat in den menschlichen Körper innerhalb von bis zu 24 Stunden resorbiert werden kann.

3. Kollagenschwamm nach Anspruch 1 oder 2, worin das Kollagen Typ I-Kollagen umfaßt.

4. Verfahren zur Herstellung eines in Anspruch 1 definierten Kollagenschwammes, wobei man eine Lösung oder Suspension aus Kollagenfasern in einer wässrigen Lösung aus Taurolidin oder Taurultam lyophilisiert.

5. Verwendung eines in Anspruch 1 definierten Kollagenschwammes in der Chirurgie.

## Revendications

1. Eponge de collagène lyophilisé pour utilisation comme implant pour les ostéites et autres cavités de l'os, ladite éponge ayant, dispersées en son sein, des quantités efficaces à effet antibactérien de taurolidine et/ou taurultame.

2. Eponge de collagène selon la revendication 1, caractérisée en ce que le collagène est choisi parmi du collagène acide soluble et du collagène neutre soluble vieilli artificiellement, afin que l'implant qui en résulte soit capable de se résorber à l'intérieur du corps humain en une durée allant jusqu'à 12 heures.

3. Eponge de collagène selon la revendication 1 ou la revendication 2, caractérisée en ce que le collagène comprend du collagène de type I.

4. Procédé pour préparer une éponge de collagène selon la revendication 1, dans lequel une solution ou suspension de fibres de collagène est lyophilisée dans une solution aqueuse de taurolidine ou taurultame.

5. Utilisation d'éponge de collagène selon la revendication 1 en chirurgie.
